# EUROPEAN PATENT SPECIFICATION

(11) **EP 1 833 421 B1**
(45) Date of publication and mention of the grant of the patent: **26.06.2013**
(21) Application number: 05855917.0
(22) Date of filing: 30.12.2005
(51) Int. Cl.: A61F 2/06, B29C 55/00, B29C 35/02, A61F 2/00

(54) **SINTERED STRUCTURES FOR VASCULAR GRAFTS**
GESINTERTE STRUKTUREN FÜR GEFÄSSPROTHESEN
STRUCTURES FRITTEES POUR GREFFONS VASCULAIRES

(30) Priority: 31.12.2004 US 26609
(43) Date of publication of application: 19.09.2007
(73) Proprietor: Boston Scientific Limited, Hastings Christ Church (BB)
(72) Inventor: HENDERSON, Jamie, Oakland, NJ 07436 (US)
(74) Representative: Vossius & Partner
(86) International application number: PCT/US2005/047428
(87) International publication number: WO 2006/074068

(56) References cited:
- WO-A-98/26731
- US-A- 4 822 341
- US-A- 5 628 786
- US-A- 5 843 171

## Description

The present invention relates to sintered structures for a vascular graft and, more specifically, to a vascular graft having a PTFE tube structure one or more discrete portions of which are sintered prior to expansion thereof such that such expansion of the PTFE tube structure results in different microstructures thereof at various locations on the PTFE tube structure.

It is well known to use extruded tube structures of polytetrafluoroethylene (PTFE) as implantable intraluminal prostheses, particularly vascular grafts. PTFE is particularly suitable as an implantable prosthesis as it exhibits superior biocompatibility. PTFE tube structures may be used as vascular grafts in the replacement or repair of a blood vessel as PTFE exhibits low thrombogenicity. In vascular applications, the grafts are manufactured from expanded polytetrafluoroethylene (ePTFE) tube structures. These tube structures have a microporous structure which allows natural tissue ingrowth and cell endothelization once implanted in the vascular system. This contributes to long term healing and patency of the graft. Grafts formed of ePTFE have a fibrous state which is defined by the interspaced nodes interconnected by elongated fibrils. [-insert new page 1a-]

A vascular graft is frequently subjected to different conditions along its length. For example, handling of the vascular graft may result in significant bending forces at specific longitudinal positions along the graft which may cause kinking of the graft. Another example of different physical forces applied to one or more specific longitudinal sections of the graft is that the graft may be punctured, such as for passage of a suture through the graft which may be for securing the graft to the tissue of the patient. Such puncturing is desirably limited to the site of the puncture to prevent tearing of the graft, which may be longitudinal, from the site of the puncture. The changes in the conditions to which the graft is subjected may occur at specific longitudinal positions on the graft, such as the puncturing thereof for a suture, or

US 5,843,171 discloses a tube of porous PTFE having at least two first and at least two second regions wherein the at least two first regions have a greater density than the at least two second regions.

US 5,628,786 discloses a microporous polytetrafluoroethylene ("PTFE") endovascular graft which has a reinforcing structure integrally bound to the graft which permits radial expansion of the graft and stabilizes the graft against longitudinal compression upon application of an axial force thereto and against axial foreshortening upon radial expansion of the graft.

US 4,822,341 relates to a vascular access fistula which includes a smooth, continuous PTFE tube having a hard sintered tube section integrally joined at its opposing ends with expanded PTFE tube sections. Inlet and outlet access port holes are formed in the hard sintered tube section to provide acute access to the fistula.

more gradually along the length of the graft, such as a bending force gradually applied thereto.

The performance of the vascular graft when subjected to various conditions depends upon the physical characteristics of a vascular graft. The physical characteristics which provide desirable performance typically differ depending on the conditions. For example, a vascular graft which has a high compressive strength will typically require higher bending forces to cause kinking of the graft. However, a graft which has such a high compressive strength uniformly throughout the length thereof may have limited transverse flexibility. Such transverse flexibility is typically desired to facilitate conformance of the graft with a lumen which has curves and bends in the body.

A vascular graft which is integral and of the same extrudate frequently has physical characteristics which are generally uniform longitudinally and transversely relative to the graft. Such vascular grafts may have satisfactory performance when subjected to certain conditions. However, the performance of such vascular grafts when subjected to a variety of conditions is typically limited.

In an effort to provide different physical characteristics to a vascular graft, separately formed structures may be bonded to an integral graft. For example, in applications where kinking is likely, vascular grafts have an additional support structure to prevent kinking. Typically, external support structures, such as helical coils, are bonded around the outer wall surface of the ePTFE tube structure. Alternatively, individual rings may be bonded to the outer wall surface of the ePTFE by injection molding.

Such additional support structures have several disadvantages. For example, the additional support structures are normally bonded to the outer wall surface of the ePTFE tube structure thereby increasing the outer diameter of the graft in the regions of the support structures. As a result, implantation of the graft can become more difficult. For example, when tunneling through tissue is required to implant the graft, such as in vascular access applications, a larger cross-sectional tunnel area is required to allow for insertion of the graft.

Another disadvantage of grafts having added support structures is that they are often made from materials which are different from the material of the graft wall and require added processing steps such as heat bonding or additional materials such as adhesive to adhere the support structure to the graft. Differential shrinkage or expansion of the external support structure relative to the ePTFE tube structure can cause the bond to weaken and/or the graft to twist significantly. Separation of the support structure from the graft is obviously undesirable.

Other ePTFE grafts have included external polymeric ribs which provide radial support to the lumen, but increase the outer diameter and wall thickness of the graft.

### Summary of the Invention

The invention is defined by the features of the claims. In particular, the vascular graft of the present invention is for implantation within a body and has a PTFE tube structure including a length and inner and outer wall surfaces. The tube structure has a non-expanded portion formed from sintering a PTFE green tube extrudate and an expanded portion formed subsequent to the sintering. The expanded and non-expanded portions are of the same extrudate. The expanded portion has a region which adjoins the non-expanded portion as defined in claim 1, wherein a degree of expansion of the region is limited by the non-expanded portion. The limiting of the expansion by the non-expanded portion is attenuated at a location of the region which is remote from the non-expanded portion. The present invention also relates to a method for making the vascular graft which facilitates the formation of the non-expanded and expanded portions of the PTFE tube structure as defined in independent claim 23.

The limitation of the degree of expansion of the expanded region which adjoins the non-expanded region and the attenuation of the limitation at a location which is remote from the non-expanded portion provides the graft with different physical characteristics at different locations thereof. Consequently, different locations of the vascular graft may be provided with specific physical characteristics which provide improved performance for the specific conditions to which the various locations of the vascular graft may be subjected. This improves the performance of the entire vascular graft by providing for the tailoring of the physical characteristics of the vascular graft to match the different conditions to which different locations of the graft may be subjected. Since a vascular graft is frequently subjected to different conditions within the body of a patient, varying the physical characteristics of the vascular graft to provide the desired performance thereof for the respective conditions will improve the overall performance of the vascular graft within the body.

Further variation in the physical characteristics of the vascular graft is provided by the non-expanded portion thereof. The non-expanded portion is typically harder and stiffer than the expanded portion which provides the vascular graft with further variation in the physical characteristics thereof. This enables the formation of a vascular graft with at least three regions of differing physical characteristics which include the non-expanded portion, the region of the expanded portion which adjoins the non-expanded portion, and the region of the expanded portion which is remote from the non-expanded portion.

The vascular graft may have more than three regions which have different physical characteristics. This may be provided, for example, by having more than one non-expanded region and by varying the shape and orientation of one or more of the non-expanded regions relative to the tube structure. Additionally, the transitions between the regions of the vascular graft which have different physical characteristics may vary. For example, the transitions may be gradual which may establish a gradient between the regions having different physical characteristics. Alternatively, the transitions between the regions may be defined by discrete boundaries which provide distinct demarcations between the regions having different physical characteristics.

These and other features of the invention as defined by the claims will be more fully understood from the following description of specific embodiments of the invention taken together with the accompanying drawings.

### Brief Description of the Drawings

In the drawings:
Fig. 1 is a side elevation view in schematic of a vascular graft of the present invention, the graft being shown as having an expanded first longitudinal region containing longitudinal non-expanded portions, and an expanded second longitudinal region;
Fig. 2 is an enlarged cross-sectional view of the vascular graft of Fig. 1 in the plane indicated by line 1-1 of Fig. 1, showing the angular positions of the non-expanded portions;
Fig. 3 is a block diagram of a method of the present invention for making the vascular graft of Fig. 1, the diagram showing schematic illustrations of the vascular graft formed by the respective steps of the method;
Fig. 4 is a side elevation view in schematic of an alternative embodiment of the vascular graft of Fig. 1, the graft being shown as having regions which have different densities;
Fig. 5 is a side elevation view in schematic of alternative embodiments of further non-expanded portions, the non-expanded portions being formed in a PTFE tube structure of a vascular graft;
Fig. 6 is an enlarged cross-sectional view of the vascular graft of Fig. 5 in the plane indicated by line 6-6 of Fig. 5, showing the angular positions of the non-expanded portions;
Fig. 7 is an enlarged cross-sectional view of the vascular graft of Fig. 5 in the plane indicated by line 7-7 of Fig. 5, showing the angular positions of the non-expanded portions;
Fig. 8 is an enlarged cross-sectional view of the vascular graft of Fig. 5 in the plane indicated by line 8-8 of Fig. 5 showing the angular positions of the non-expanded portions;
Fig. 9 is an enlarged side elevation view in schematic of a portion of an alternative embodiment of the vascular graft of Fig. 1 showing the inclined orientation of the nodes of the PTFE microstructure of the graft;
Fig. 10 is an enlarged cross-sectional view of the portion of the vascular graft of Fig. 9 in the plane indicated by line 10-10 of Fig. 9, showing the angular positions of the non-expanded portions;
Fig. 11 is a side elevation view in schematic of a PTFE green tube extrudate from which the vascular graft of Fig. 9 may be formed, the extrudate being shown as having longitudinal pre-sintered portions which are longitudinally offset; and
Fig. 12 is an enlarged side elevation view in schematic of a portion of a vascular graft showing the vertical orientation of the nodes of the PTFE microstructure of the graft.

Corresponding reference characters indicate corresponding parts throughout the several views of the drawings.

### Detailed Description of the Invention

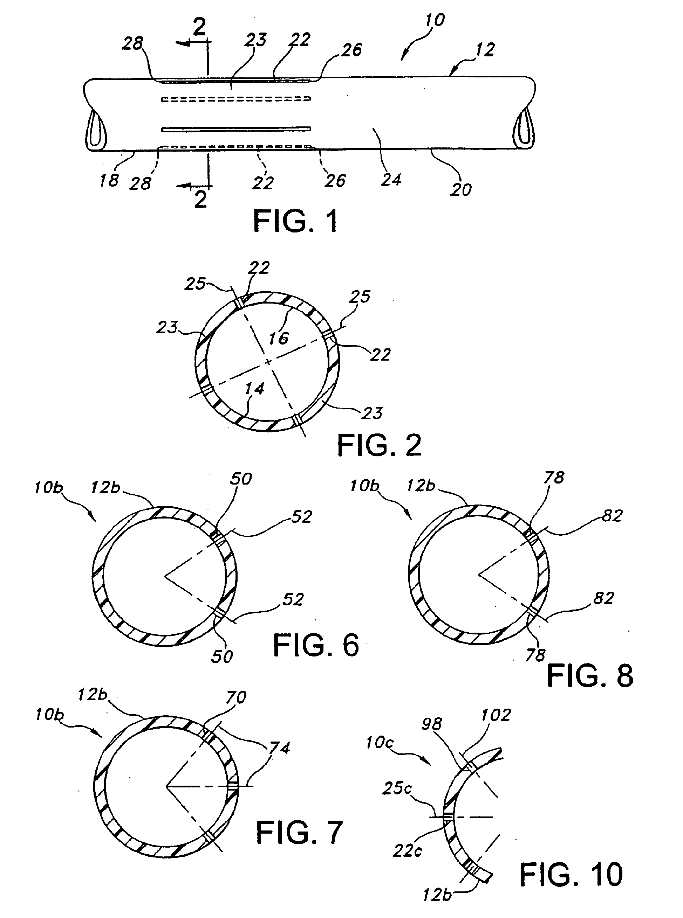

Referring to the drawings and more particularly to Fig. 1, a vascular graft 10 is shown as including a tube structure 12 having a length and inner and outer wall surfaces 14, 16. The tube structure 12 is formed of polytetrafluoroethylene (PTFE) material.

The tube structure 12 includes first and second longitudinal sections 18, 20. The first longitudinal section 18 includes four non-expanded portions 22 formed from sintering a PTFE green tube extrudate. The region of the first longitudinal section 18, which is not included in the non-expanded portions 22, is expanded such that the first longitudinal section has an expanded portion 23 in addition to the non-expanded portions 22. The second longitudinal section 20 is expanded such that it constitutes another expanded portion 24.

The non-expanded portions 22 are each elongate and have a longitudinal central axis which is contained in a corresponding longitudinal cross-sectional plane 25 of the PTFE tube structure 12. The non-expanded and expanded portions 22, 23, 24 are of the same extrudate.

Adjacent pairs of the non-expanded portions 22 are separated from one another circumferentially relative to the PTFE tube structure 12 by an angular dimension equal to 90 degrees, as shown in Fig. 2. The non-expanded portions 22 each have respective proximal and distal ends 26, 28. The proximal and distal ends 26, 28 have the same respective longitudinal positions relative to the PTFE tube structure 12, as shown in Fig. 1.

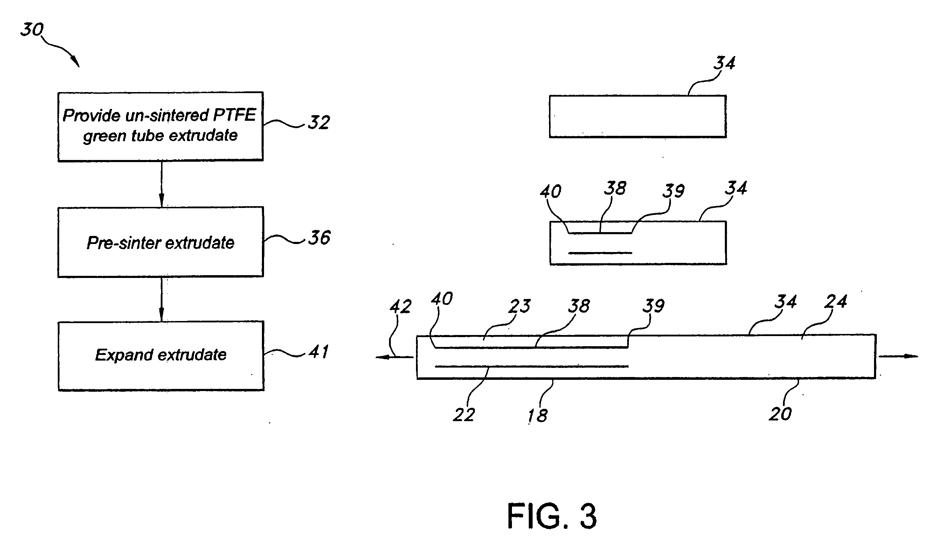

The vascular graft 10 may be formed according to the method 30 shown in Fig. 3. The method 30 includes providing 32 a PTFE green tube extrudate 34 which is un-sintered. Following the providing step 32, the method 30 includes a pre-sintering step 36 during which discrete portions 38 of the PTFE green tube extrudate 34 are sintered. The pre-sintering step 36 provides for the sintering of discrete portions 38 of the extrudate 34. The such discrete portions 38 may be elongate and have a longitudinal central axis which is contained in a respective longitudinal cross-sectional plane which corresponds to the longitudinal cross-sectional planes 25 shown in Fig. 2. The discrete portions 38 have proximal and distal ends 39, 40 which have the same respective longitudinal positions relative to the PTFE tube structure 12, as shown in Fig. 3. The pre-sintering 36 locks the microstructure of the discrete portions 38 so that the microstructure thereof is the same as the microstructure of the extrudate 34.

Following the pre-sintering step 36, the method 30 includes an expansion step 41 during which a uniform longitudinal tensile force 42 is applied to the extrudate 34. The application of the tensile force 42 produces expansion of the extrudate 34 and longitudinal elongation of the portions thereof which are not pre-sintered. Such expansion produces a node and fibril microstructure in the regions of the extrudate 34 which are expanded. Consequently, the expanded regions of the extrudate 34 constitute the expanded portions 23, 24 and the pre-sintered discrete portions 38 constitute the non-expanded portions 22.

The application of the tensile force 42 produces longitudinal elongation of the non-expanded portions 22 and the expanded portions 23, 24. The microstructure of the non-expanded portions 22 resist elongation to a greater degree than the microstructure of the expanded portions 23, 24. Consequently, the non-expanded portions 22 restrict the elongation of the regions of the expanded portions 23 in close proximity to the non-expanded portions, because the non-expanded and expanded portions are integral with one another as a result of being of the same extrudate 34. Consequently, the elongation of the expanded portion 23 is limited because of the longitudinal position thereof relative to the extrudate 34 being the same as the longitudinal position of the non-expanded portions 22 relative to the extrudate. The elongation of the expanded portion 24 is not significantly limited by the non-expanded portions 22 because of the different longitudinal positions thereof relative to the extrudate. Consequently, the elongation of the first longitudinal section 18, which contains non-expanded and expanded portions 22, 23, is less than the elongation of the second longitudinal section 20, which does not contain any of the non-expanded portions, where such elongation results from the application of a longitudinal tensile force 42 to the extrudate 34, including the first and second longitudinal sections 18, 20, after the pre-sintering step 36. In a preferred embodiment, the elongations of the first and second longitudinal sections 18, 20 are 200% and 800%, respectively. Alternatively, if the first and second longitudinal sections 18, 20 are to be elongated by generally the same amount, then a slightly greater tensile force is required to be applied to the first longitudinal section as compared to the tensile force applied to the second longitudinal section. Additionally, the elongation of the first longitudinal section 18 may be varied by changing the fraction of the cross-section area thereof which is constituted by the non-expanded portions 22. The amount of the cross-sectional area of the first longitudinal section 18 constituted by the non-expanded portions 22 may be varied by changing the number or transverse dimension of the non-expanded portions.

The first and second longitudinal sections 18, 20 are each expanded where the degree of expansion of the first longitudinal section is less than the degree of expansion of the second longitudinal section. The respective degrees of expansion of the first and second longitudinal sections 18, 20 correspond to the respective longitudinal elongations thereof. The reduced degree of expansion of the first longitudinal section 18 relative to the second longitudinal section 20 results from the first longitudinal section containing the non-expanded portions 22. The non-expanded portions 22 limit the degree of expansion of the region of the PTFE tube structure 12 which adjoins the non-expanded portions. This limiting of the degree of expansion becomes increasingly attenuated at locations of the region of the PTFE tube structure 12 which are increasingly remote from the non-expanded portions 22. Consequently, the degree of expansion of the second longitudinal section 20 is not significantly affected by the non-expanded portions 22.

The reduced longitudinal elongation of the first longitudinal section 18 can be controlled by varying the number, width and location of the non-expanded portions 22 relative to the PTFE tube structure 12. Consequently, the magnitudes of the longitudinal elongations of the first and second longitudinal sections 18, 20 resulting from the same longitudinal tensile force may be optimized. The longitudinal elongation of the PTFE tube structure 12 is related to the density thereof such that the density may be controlled by control of such elongation. Additionally, different portions of the PTFE tube structure 12 may be formed to have different densities by controllably varying the longitudinal elongation of the corresponding portions. In a preferred embodiment, the first longitudinal section 18 is elongated by 200% and the second longitudinal section 20 is elongated by 800%.

The relative elongations of the first and second longitudinal sections 18, 20 may be varied by altering the rate at which the longitudinal tensile force 42 is applied to the extrudate 34. For example, applying the force 42 at a sufficiently rapid rate may result in the elongations of the first and second longitudinal sections 18, 20 being 400% and 600%, respectively. Alternatively, applying the force 42 at a sufficiently slow rate may result in the elongations of the first and second longitudinal sections 18, 20 being 0% and 1000%, respectively.

Alternative embodiments of the vascular graft 10 have one or more further non-expanded portions which have shapes, dimensions, and locations relative to the tube structure 12 which differ from the non-expanded portions 22 shown in Figs. 1 and 2. Such alternative embodiments of the vascular graft 10 may be made according to the method 30 except that the pre-sintering step 36 may be performed on a portion of the extrudate 34 which has a shape, dimension, and location which differs from the discrete portions 38 shown in Fig. 3. The one or more non-expanded portions of such a vascular graft may be located relative to the tube structure 12 in adjoining relation to one or more regions of expanded portions which correspond to the expanded portions 23 shown in Fig. 1. Such adjoining relation results in the elongation of the one or more regions of the expanded portions being limited by the one or more adjoining non-expanded portions. The limiting of the elongation by the one or more non-expanded portions is attenuated at a location of the region which is remote from the non-expanded portion. An example of such a region which is sufficiently remote from the non-expanded portion such that the limiting of the elongation is attenuated is the second longitudinal section 20. This remoteness results in the elongation of the second longitudinal section 20 not being significantly limited by the non-expanded portions 22.

The shape, dimensions and location relative to the tube structure 12 of the one or more non-expanded portions may be selected such that the limiting of the elongation of the non-expanded portions by the non-expanded portions is increasingly attenuated at locations of the region which are increasingly remote from the non-expanded portion. This may provide a gradient of elongation of the expanded portion in which the elongation gradually increases in regions of the expanded portion which are increasingly remote from the non-expanded portion.

Expansion of the portion of the first longitudinal section 18 which does not contain the non-expanded portions, and expansion of the second longitudinal section 20 produces expanded portions 23 which have node and fibril microstructures. This microstructure differs from the microstructure of the non-expanded portions 22 which is the same as the microstructure of the PTFE green tube extrudate. The difference in the microstructures of the non-expanded and expanded portions 22, 23 results in differences in the physical characteristics thereof. For example, if a sufficiently large longitudinal tensile force is applied to the tube structure 12, the length of the non-expanded portions 22 will increase while the cross-sectional area thereof will decrease. This combination of changes in the dimensions of the non-expanded portions 22 is sometimes referred to as "necking down" of the non-expanded portions. In contrast, application of a longitudinal tensile force to the expanded portions 23 will cause an increase in the length thereof but the cross-sectional area of the expanded portions will remain essentially the same, although an insignificant decrease in the cross-sectional area is possible. Additionally, application of such a longitudinal tensile force to the expanded portions 23 will cause a decrease in the density and an increase in the porosity of the expanded portions.

Another difference in the physical characteristics of the non-expanded and expanded portions 22, 23 is that application of the same longitudinal tensile force to non-expanded and expanded portions having the same dimensions will normally produce a smaller increase in the length of the non-expanded portion as compared to the length of the expanded portion. However, rapidly applying the longitudinal tensile force to the non-expanded portion 22 will produce a smaller increase in the longitudinal elongation thereof as compared to more slowly applying the force, where the maximum magnitude of the applied force is the same. A rapid application of the longitudinal tensile force may result from reducing the time duration between the initial application of the force and the full magnitude of the force. Increasing this time duration provides a slower application of the force. In contrast, the respective elongations of the expanded portion 23 produced by rapid and slower applications of the longitudinal tensile force thereto are as compared to the differences in the elongation of the non-the expanded portion 22 resulting from the rapid and slower force applications. Consequently, as the speed with which the longitudinal tensile force is applied decreases, the increase in the length of the non-expanded portion 22 becomes closer to the increase in the length of the expanded portion 23.

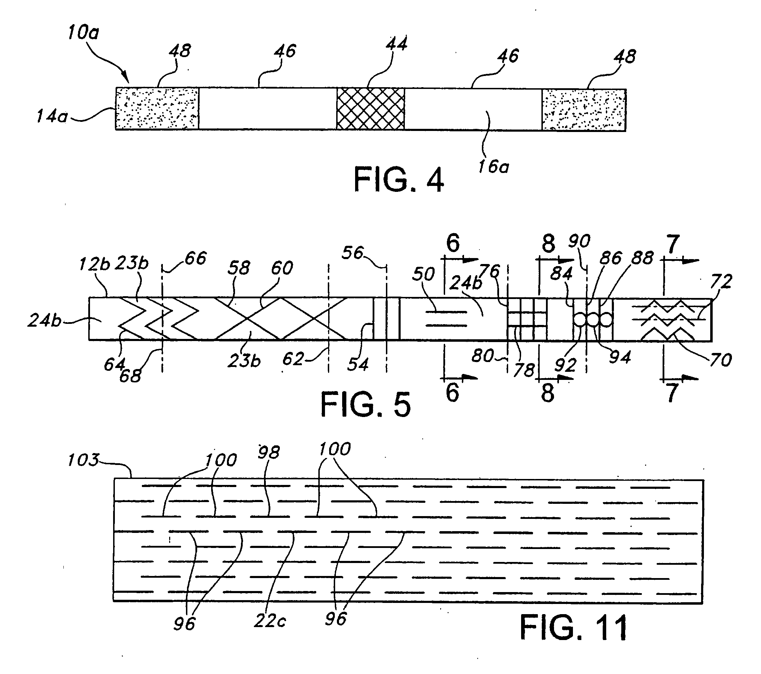

The limitation on the expansion of the regions of the expanded portions 23, 24 which are sufficiently near the non-expanded portions 22 may provide for the controlled variation in the physical characteristics of the tube structure 12. For example, limiting the elongation of the expanded portions 23, 24 limits the decrease in density thereof which normally results from elongation of the expanded portions. Consequently, forming the tube structure 12 such that the expanded portions 23, 24 have regions with different amounts of elongation provides the corresponding regions to have different densities. This is illustrated in Fig. 4 which shows a schematic view of an alternative second embodiment of the vascular graft 10a. The vascular graft 10a includes a tube structure 12a and has inner and outer wall surfaces 14a, 16a. In these and additional respects, the vascular graft 10a corresponds to the vascular graft 10. Accordingly, parts illustrated in Fig. 4 which correspond to parts illustrated in Figs. 1 and 2 have, in Fig. 4, the same reference numeral as in Figs. 1 and 2, with the addition of the suffix "a". The vascular graft 10a has an inner expanded portion 44 and intermediate and outer expanded portions 46, 48 located proximally and distally of the inner expanded portion. The inner and outer expanded portions 44, 48 each have non-expanded portions 22a. The amount of non-expanded portions 22a in the inner expanded portion 44 is greater than the amounts of non-expanded portions 22a in either of the outer expanded portion 48. The intermediate expanded portions 46 do not have non-expanded portions 22a. Consequently, the intermediate expanded portions 46 each have a standard graft density. The inner expanded portion 44 has a high density. The outer expanded portions 48 each have a moderate density. The respective densities of the inner expanded portion 44 and the intermediate and outer expanded portions 46, 48 results in the respective portions being particularly suitable for different applications. For example, the high density of the inner expanded portion 44 results in a high suitability thereof for support replacement, such as providing for replacement of a conventional stent which may be secured to the tube structure 12a, and the associated support provided by such a stent. Also, the high density of the inner expanded portion 44 provides for high suitability thereof for use in a high wear zone. The moderate densities of the outer expanded portions 48 result in a high suitability thereof for suturing or attachment.

Alternative embodiments of further non-expanded portions 50, 54, 58, 60, 64, 70, 76, 78, 84, 86, 88, 92, 94 of Figs. 1 and 2 are shown in Fig. 5. Fig. 5 is a side elevation view in schematic of a vascular graft 10b including a PTFE tube structure 12b in which the non-expanded portions 50, 54, 58, 60, 64, 70, 76, 78, 84, 86, 88, 92, 94 are formed. The non-expanded portions 50, 54, 58, 60, 64, 70, 76, 78, 84, 86, 88, 92, 94 may be formed according to a method which corresponds in some respects to the method 30. In these and additional respects, the vascular graft 10b corresponds to the vascular graft 10. Accordingly, parts illustrated in Fig. 5 which correspond to parts illustrated in Figs. 1 and 2 have, in Fig. 5, the same reference numeral as in Figs. 1 and 2, with the addition of the suffix "b".

One or more of the non-expanded portions 50 may be formed in the tube structure 12b. Each of the non-expanded portions 50 is formed from sintering the PTFE green tube extrudate, such that the non-expanded portions and expanded portions 23b, 24b are of the same extrudate. Each of the non-expanded portions 50 is elongate and has a longitudinal axis which is contained in a longitudinal cross-sectional plane 52 of the PTFE tube structure 12b. In these respects, the non-expanded portions 50 correspond to the non-expanded portions 22 shown in Figs. 1 and 2. Each of the two non-expanded portions 50 shown in Fig. 5 has a proximal and distal end which may have the same or different longitudinal positions relative to the tube structure 12b. Additionally, the circumferential spacing of the two or more of the non-expanded portions 50 may be uniform or different.

One or more of the non-expanded portions 54 may be formed in the tube structure 12b. Each of the non-expanded portions 54 is formed from sintering the PTFE green tube extrudate, such that the non-expanded portions and expanded portions 23b, 24b are of the same extrudate. Each of the non-expanded portions 54 is elongate and has a longitudinal axis which is contained in a transverse cross-sectional plane 56 of the PTFE tube structure 12b. One or more of the non-expanded portions 54 may encircle the inner wall surface 14b such that these non-expanded portions are annular.

One or more of the first and second non-expanded portions 58, 60 may be formed in the tube structure 12b. Each of the first and second non-expanded portions 58, 60 is formed from sintering the PTFE green tube extrudate, such that the non-expanded portions and expanded portions 23b, 24b are of the same extrudate. Each of the first and second non-expanded portions 58, 60 is elongate and has a longitudinal central axis which is inclined relative to a transverse cross-sectional plane 62 of the PTFE tube structure 12b. The first and second non-expanded portions 58, 60 have opposite inclinations and intersect one another, as shown in Fig. 5.

One or more of the non-expanded portions 64 may be formed in the tube structure 12b. Each of the non-expanded portions 64 is formed from sintering the PTFE green tube extrudate, such that the non-expanded portions and expanded portions 23b, 24b are of the same extrudate. Each of the non-expanded portions 64 has an elongate saw-tooth configuration, and a longitudinal principal axis 66 which bisects the saw-tooth configuration. The principal axis 66 is contained in a transverse cross-sectional plane 68 of the PTFE tube structure 12b.

One or more of the non-expanded portions 70 may be formed in the tube structure 12b. Each of the non-expanded portions 70 is formed from sintering the PTFE green tube extrudate, such that the non-expanded portions and expanded portions 23b, 24b are of the same extrudate. Each of the non-expanded portions 70 has an elongate saw-tooth configuration, and a longitudinal principal axis 72 which bisects the saw-tooth configuration. The principal axis 72 is contained in a longitudinal cross-sectional plane 74 of the PTFE tube structure 12b.

Two or more of the transverse non-expanded portions 76, and two or more of the longitudinal non-expanded portions 78, may be formed in the tube structure 12b. Each of the non-expanded portions 76, 78 is formed from sintering the PTFE green tube extrudate, such that the non-expanded portions and expanded portions 23b, 24b are of the same extrudate.

The transverse non-expanded portions 76 each are elongate and have a longitudinal central axis which is contained in a corresponding transverse cross-sectional plane 80 of the PTFE tube structure 12b. The transverse non-expanded portions 76 are separated from one another longitudinally relative to the PTFE tube structure 12b.

The longitudinal non-expanded portions 78 each are elongate and have a longitudinal central axis which is contained in a corresponding longitudinal cross-sectional plane 82 of the PTFE tube structure 12b. The longitudinal non-expanded portions 78 are separated from one another transversely relative to the PTFE tube structure 12b.

The longitudinal non-expanded portions 78 intersect the transverse non-expanded portions 76, as shown in Fig. 5. More than two transverse non-expanded portions 76 may intersect the longitudinal non-expanded portions 78, as shown in Fig. 5.

A first, second and third transverse non-expanded portions 84, 86, 88 may be formed in the tube structure 12b. Each of the non-expanded portions 84, 86, 88 is formed from sintering the PTFE green tube extrudate, such that the non-expanded portions and expanded portions 23b, 24b are of the same extrudate. Each of the non-expanded portions 84, 86, 88 is elongate and has a longitudinal axis which is contained in a transverse cross-sectional plane 90 of the PTFE tube structure 12b. One or more of the non-expanded portions 84, 86, 88 may encircle the inner wall surface 14b such that these non-expanded portions are annular.

A first and second annular non-expanded portions 92, 94 may be formed in the tube structure 12b. Each of the non-expanded portions 92, 94 is formed from sintering the PTFE green tube extrudate, such that the non-expanded portions and expanded portions 23b, 24b are of the same extrudate. The first annular non-expanded portion 92 is located between the first and second transverse non-expanded portions 84, 86 in tangential relation thereto, as shown in Fig. 5. The second annular non-expanded portion 94 is located between the second and third transverse non-expanded portions 86, 88 in tangential relation thereto. Additional transverse non-expanded portions and annular non-expanded portions in tangential relation thereto are possible, as shown in Fig. 5.

The vascular graft 10b may have one or more non-expanded portions formed from sintering the PTFE green tube extrudate, such that the one or more non-expanded portions and expanded portions 23b, 24b are of the same extrudate, and the one or more non-expanded portions have the configuration of a lattice structure.

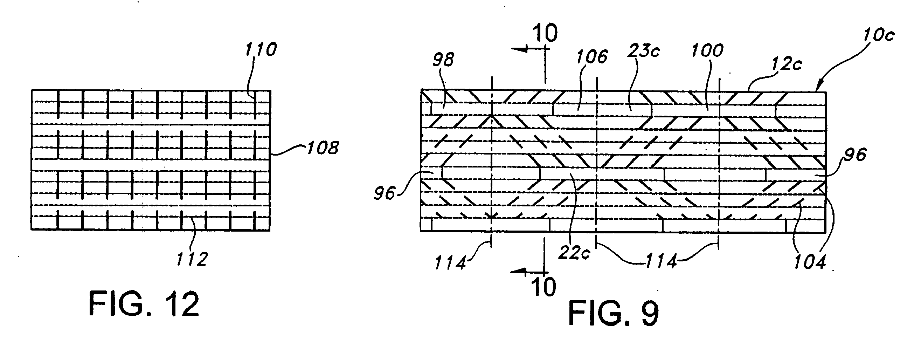

Alternative embodiments of the non-expanded portions 22 of Figs. 1 and 2 are shown in Figs. 9 and 10. Fig. 9 shows a portion of a vascular graft 10c including a PTFE tube structure 12c in which the non-expanded portions 22c, 96, 98, 100 are formed. The non-expanded portions 22c, 96, 98, 100 may be formed according to a method which corresponds in some respects to the method 30. In these and additional respects, the vascular graft 10c corresponds to the vascular graft 10. Accordingly, parts illustrated in Fig. 5 which correspond to parts illustrated in Figs.1 and 2 have, in Fig. 5, the same reference numeral as in Figs. 1 and 2, with the addition of the suffix "c".

The non-expanded portions 22c, 96 are designated herein as the first non-expanded portion 22c and first supplemental non-expanded portions 96. The non-expanded portions 22c, 96 are each elongate and have a longitudinal central axis which is contained in a first longitudinal cross-sectional plane 25c of the PTFE tube structure, as shown in Fig. 10. The non-expanded portions 98, 100 are designated herein as the second non-expanded portion 98 and second supplemental non-expanded portions 100. The non-expanded portions 98, 100 are each elongate and have a longitudinal central axis which is contained in a second longitudinal cross-sectional plane 102 of the PTFE tube structure. The first and first supplemental non-expanded portions 22c, 96 are separated from the second and second supplemental non-expanded portions 98, 100 circumferentially relative to the PTFE tube structure 12c.

The first and first supplemental non-expanded portions 22c, 96 have the same longitudinal dimension and are separated longitudinally from adjacent ones of the first supplemental and first non-expanded portions by uniform dimensions. The second and second supplemental non-expanded portions 98, 100 have the same longitudinal dimension and are separated longitudinally from adjacent ones of the second supplemental and second non-expanded portions by uniform dimensions.

The first and second non-expanded portions 22c, 98 and the first and second supplemental non-expanded portions 96, 100 are each formed from sintering the PTFE green tube extrudate. Fig. 11 shows the PTFE green tube extrudate 103 after the formation of the non-expanded portions 22c, 96, 98, 100 and before the formation of the expanded portion 23c. Before the formation of the expanded portion 23c, the differences between the longitudinal positions of the first and second non-expanded portions 22c, 98 and between the corresponding pairs of the first and second supplemental non-expanded portions 96, 100 are the same, as shown in Fig. 11.

The expanded portion 23c is formed from longitudinally elongating the PTFE green tube extrudate 103 in which the non-expanded portions 22c, 96, 98, 100 have been previously formed. The first and second non-expanded portions 22c, 98, the first and second supplemental non-expanded portions 96, 100, and the expanded portions 23c are of the same extrudate 103. The expanded portion 23c corresponds to the expanded portion 23 in that the microstructures of such expanded portions are affected by the respective proximities thereof to the non-expanded portions 22c, 96, 98, 100, 22, as described further hereinbelow.

The elongation of the PTFE green tube extrudate 103 which provides for the formation of the expanded portion 23c also causes the first and second non-expanded portions 22c, 98 to be longitudinally displaced relative to one another. This longitudinal displacement between corresponding pairs of the non-expanded portions, such as the first and second non-expanded portions 22c, 98, is referred to herein as the longitudinal offset thereof. The longitudinal offset may provide for parts of corresponding pairs of the non-expanded portions, such as the first and second non-expanded portions 22c, 98, to have the same longitudinal position relative to the tube structure 12c, and other parts of the corresponding pairs of the non-expanded portions to have different longitudinal positions, as shown in Fig. 9. Such relative longitudinal positions of corresponding pairs of the non-expanding portions in which parts thereof have the same longitudinal positions and other parts of the non-expanded portions have different longitudinal positions is referred to herein as partial longitudinal overlap, which is illustrated, for example, by the first and second non-expanded portions 22c, 98 in Fig. 9.

The uniformity of the differences between the longitudinal positions of the corresponding pairs of the non-expanded portions 22c, 96, 98, 100 in the green tube extrudate 103 results in a uniform longitudinal separation between the first and first supplemental non-expanded portions 22c, 96 and between the second and second supplemental non-expanded portions 98, 100 in the tube structure 12c. Additionally, after the elongation of the green tube extrudate 103, the differences between the longitudinal positions of the corresponding pairs of the non-expanded portions 22c, 96, 98, 100 are the same, as shown in Fig. 9.

The relative longitudinal displacement between the first and second non-expanded portions 22c, 98 affects the node and fibril microstructure of the expanded portion 23c which includes nodes 104 and fibrils 106. More specifically, the nodes 104 thereof extend between the first and second non-expanded portions 22c, 98, as shown in Fig. 9. The relative longitudinal displacement between the first and second non-expanded portions 22c, 98 causes the nodes 104 to have an inclined orientation relative to a longitudinal cross-sectional plane 25c of the PTFE tube structure 12c subsequent to the formation of the expanded portion 23c. The orientation of the nodes 140 may also be considered as skewed or angular. The correspondence between the longitudinal offset of the non-expanded portions 22c, 96, 98, 100 also results in the inclinations of the nodes 104 between the first and first supplemental non-expanded portions 22c, 96 and an inclination of the nodes 104 between the second and second supplemental non-expanded portions 98, 100. The respective inclinations of the nodes 104 between adjacent pairs of the non-expanded portions 22c, 96, 98, 100 are symmetrical about the transverse cross-sectional planes 114 of the PTFE tube structure 12c.

The inclinations of the nodes 104 enable the tube structure 12c to be radially compressed when the tube structure is subjected to a sufficiently large transverse force. Such radial compression may result in the transverse dimension of the cross-section of the tube structure 12c being reduced and the shape of the cross-section remaining constant. Consequently, a tube structure 12c which is circular may remain circular during a radial compression thereof with the diameter of the cross-section being reduced as a result of the radial compression. Also, folding of the wall of the tube structure 12c is not necessary. Reducing the transverse dimension of the cross-section of the tube structure 12c may facilitate insertion of the graft 10c into the body of a patient. Alternatively, the inclinations of the nodes 104 may result in the tube structure 12c collapsing transversely into an elliptical or flat cross-sectional configuration when subjected to a sufficiently large transverse force. Such elliptical or flat collapsing of the tube structure 12c may be accompanied by a reduction in one or more transverse dimensions of the tube structure 12c. Collapsing of the cross-section of the tube structure 12c, with or without reduction in one or more of the transverse dimensions, may facilitate insertion of the graft 10c into the body of a patient.

To further illustrate by way of comparison the inclined nodes of the microstructure shown in Fig. 9, a PTFE tube structure 108 having a node and fibril microstructure is shown in Fig. 12. The node and fibril microstructure shown in Fig. 12 is typically formed from the expansion of a PTFE green tube extrudate which provides the PTFE tube structure 108. Such an expansion typically results in the tube structure 108 having a microstructure including nodes 110 which have a transverse orientation relative to the tube structure, as shown in Fig. 12.

The PTFE tube structure 12c contains a substantial number of non-expanded portions 22c, 96, 98, 100, as indicated by Figs. 9 and 11. Each of the non-expanded portions 22c, 96, 98, 100 formed in the extrudate 103 shown in Fig. 11 is included as a non-expanded portion in the tube structure 12c shown in Fig. 9. While the number of non-expanded portions 22c, 96, 98, 100 shown in Figs. 9 and 11 is a preferred embodiment, fewer non-expanded portions may be formed in the tube structure 12c. Such a tube structure 12c may include an expanded portion 23c having a node and fibril microstructure in which the nodes 104 thereof have an inclined orientation as shown in Fig. 9, provided the non-expanded portions have the offset relation, such as between the non-expanded portions 22c, 98. Such a microstructure including one or more nodes 104 having the inclined orientation as shown in Fig. 9 may be provided in the tube structure 12c including as few as the first and second non-expanded portions 22c, 98.

The vascular grafts 10, 10a, 10b, 10c have different physical characteristics which result from the incorporation of the non-expanded portions in the respective tube structures 12, 12a, 12b, 12c. The differences in the physical characteristics result from differences in the positioning of the non-expanded portions relative to the respective tube structures 12, 12a, 12b, 12c. This positioning of the non-expanded portions may be defined by the orientation thereof relative to a transverse cross-sectional plane, such as the planes 62, 114 of the respective tube structures 12b, 12c. Tube structures, such as the tube structures 12, 12c, having different physical characteristics may also be provided by incorporating therein different numbers of the non-expanded portions. Differences in the number and orientation of the non-expanded portions in the respective tube structures 12, 12a, 12b, 12c may provide a corresponding resistance to compression thereof in the respective transverse cross-sectional plane, such as the planes 62, 114.

Vascular grafts are also disclosed in US2006/149361 and US2006/155371 by the same applicant.

While the invention has been described by reference to certain preferred embodiments, it should be understood that numerous changes could be made within the scope of the inventive concept described. Accordingly, it is intended that the invention not be limited to the disclosed embodiments, but that it have the full scope permitted by the language of the following claims.

## Claims

1. A vascular graft (10) for implantation within a body, said vascular graft (10) comprising
a PTFE tube structure (12) having a longitudinal length and inner (14) and outer (16) wall surfaces and first and second longitudinal sections (18, 20) at different lontitudinal positions ,
said tube structure (12) having a non-expanded portion (22) in the first longitudinal section (18) formed from sintering a PTFE green tube extrudate (34), a first expanded portion (23) in said first longitudinal section (18) and a second expanded portion (24) constituted by said second longitudinal section (20) both formed subsequent to the sintering,
said expanded and non-expanded portions (22) being of the same extrudate,
said first expanded portion (23) having a region which adjoins said non-expanded portion (22) wherein a degree of expansion of said region is limited by said non-expanded portion (22), said limiting of said expansion by said non-expanded portion (22) being attenuated at a location of said region which is remote from said non-expanded portion (22),
wherein said non-expanded portion (22) is elongate and has a longitudinal central axis contained in a longitudinal cross-sectional plane (25) of said PTFE tube structure (12).

2. A vascular graft according to claim 1, wherein said expanded portion (23, 26) has a node and fibril microstructure.

3. A vascular graft according to claim 1, wherein said non-expanded portion (22) comprises a first non-expanded portion, said vascular graft further comprising three additional non- expanded portions (22) formed from sintering the PTFE green tube extrudate (34), said first non- expanded and three additional non-expanded portions each being elongate and having a longitudinal central axis contained in respective longitudinal cross-sectional planes of said PTFE tube structure (12), said expanded (23, 24) and first and three additional non-expanded portions (22) being of the same extrudate, adjacent pairs of said first and additional non-expanded portions (22) being separated from one another circumferentially relative to said PTFE tube structure (12) by an angular dimension equal to 90 degrees, said first and additional non-expanded portions (22) each having respective proximal and distal ends (26, 28), said proximal ends having the same longitudinal positions relative to said PTFE tube structure, said distal ends having the same longitudinal position relative to said PTFE tube structure (12),
said first and additional non-expanded portions being contained within the first longitudinal section of said PTFE tube structure (12) such that application of a uniform longitudinal tensile force to said PTFE tube structure causes longitudinal elongation of said first longitudinal section and longitudinal elongation of the second longitudinal section of said PTFE tube structure (12), the longitudinal elongation of said first longitudinal section being less than the longitudinal elongation of said second longitudinal section.

4. A vascular graft according to claim 3, wherein the uniform longitudinal tensile force causes the longitudinal elongation of said first longitudinal section to be 200% and the longitudinal elongation of said second longitudinal section to be 800%.

5. A vascular graft according to claim 1 , wherein said limiting of said expansion by said non-expanded portion (22) is increasingly attenuated at locations of said section which are increasingly remote from said non-expanded portion (22).

6. A vascular graft according to claim 1 , wherein a further non-expanded portion (54) is elongate and has a longitudinal central axis contained in a transverse cross-sectional plane (56) of said PTFE tube structure.

7. A vascular graft according to claim 6, wherein said further non-expanded portion (54) encircles said inner wall surface such that said non-expanded portion (54) is annular.

8. A vascular graft according to claim 1, wherein a further non-expanded portion (58, 60) is elongate and has a longitudinal central axis which is inclined relative to a transverse cross-sectional plane (56) of said PTFE tube structure.

9. A vascular graft according to claim 8, wherein the graft comprises a first non-expanded portion (58, 60),
said vascular graft (10) comprising a second non-expanded portion formed from sintering the PTFE green tube extrudate, said expanded and second non-expanded portions being of the same extrudate,
said second non-expanded portion (60, 58) being elongate and having a longitudinal central axis which is inclined relative to a transverse cross-sectional plane (56) of said PTFE tube structure (12), said inclination of said second non-expanded portion being opposite to said inclination of said first non-expanded portion, said first and second non-expanded portions intersecting one another.

10. A vascular graft according to claim 1 , wherein a further non-expanded portion (64, 70) has an elongate saw-tooth configuration.

11. A vascular graft according to claim 10, wherein a further non-expanded portion (64) has a longitudinal principal axis (66) which bisects said saw-tooth configuration, said principal axis (66) being contained in a transverse cross-sectional plane (68) of said PTFE tube structure.

12. A vascular graft according to claim 10, wherein a further non-expanded portion (70) has a longitudinal-principal axis (72) which bisects said saw-tooth configuration, said principal axis (72) being contained in a longitudinal cross-sectional plane (74) of said PTFE tube structure.

13. A vascular graft according to claim 1 , wherein said vascular graft further comprises non-expanded portions formed from a transverse non-expanded portion (76) , said vascular graft further comprising another transverse non-expanded portion formed from sintering the PTFE green tube extrudate (34), said expanded and transverse non-expanded portions being of the same extrudate,
said transverse non-expanded portions (76) each being elongate and having a longitudinal central axis which is contained in a corresponding transverse cross-sectional plane of said PTFE tube structure, said transverse non-expanded portions (76) being separated from one another longitudinally relative to said PTFE tube structure,
wherein said longitudinal non-expanded portions (78) formed from sintering the PTFE green tube extrudate, said longitudinal non-expanded portions (78) each being elongate and having a longitudinal central axis which is contained in a corresponding longitudinal cross-sectional plane (25) of said PTFE tube structure (12), said expanded and longitudinal non-expanded portions being of the same extrudate and separated from one another transversely relative to said PTFE tube structure (12),
said longitudinal non-expanded portions (78) intersecting said transverse non-expanded portions.

14. A vascular graft according to claim 1, wherein said graft further comprises a non-expanded portion constituting a first transverse non-expanded portion (84), said vascular graft further comprising a second transverse non-expanded portion (86) formed from sintering the PTFE green tube extrudate (34),
said first and second transverse non-expanded portions (84, 86) each being elongate and having a longitudinal central axis which is contained in a corresponding transverse cross-sectional plane of the PTFE tube structure (12), said expanded and first and second transverse non-expanded portions being of the same extrudate, said first and second transverse non-expanded portions being separated from one another longitudinally relative to said PTFE tube structure,
said vascular graft (10) further comprising an annular non-expanded portion (92, 94) which is formed from sintering the PTFE green tube extrudate (34), said expanded and annular non-expanded portions being of the same extrudate, said annular non-expanded portion (92, 94) being located between said first and second transverse non-expanded portions in tangential relation thereto.

15. A vascular graft according to claim 14, wherein said annular non-expanded portion (92, 94) constitutes a first annular non-expanded portion, said vascular graft further comprising a third transverse non-expanded portion (88) formed from sintering the PTFE green tube extrudate (34), said third transverse non-expanded portion being elongate and having a longitudinal central axis which is contained in a corresponding transverse cross-sectional plane of said PTFE tube structure, said expanded and third transverse non-expanded portions being of the same extrudate, said third transverse non-expanded portion being elongate and separated from said first and second transverse non-expanded portions longitudinally relative to the PTFE tube structure such that said third transverse non-expanded portion is longitudinally separated from said first annular non-expanded portion,
said vascular graft further comprising a second annular non-expanded portion (92, 94) which is formed from sintering the PTFE green tube extrudate, said expanded and second annular non-expanded portions being of the same extrudate, said second annular non-expanded portion being located between said third transverse non-expanded portion and one of said first and second transverse non-expanded portions in tangential relation thereto.

16. A vascular graft according to claim 1, wherein said graft further comprises a non-expanded portion comprising a lattice structure.

17. A vascular graft according to claim 1, wherein said non-expanded portion (22, 96, 98, 100) constitutes a first non-expanded portion which is elongate and has a longitudinal central axis which is contained in a first longitudinal cross-sectional plane of said PTFE tube structure,
said vascular graft further comprising a second non-expanded portion formed from sintering the PTFE green tube extrudate, said second non-expanded portion being elongate and having a longitudinal central axis which is contained in a second longitudinal cross- sectional plane of said PTFE tube structure, said second non-expanded portion being separated from said first non-expanded portion circumferentially relative to said PTFE tube structure, said expanded and second non-expanded portion being of the same extrudate,
said first and second non-expanded portions having longitudinally offset positions relative to one another wherein said first and second non-expanded portions are longitudinally displaced relative to one another during the formation of said expanded portion.

18. A vascular graft according to claim 17, wherein said longitudinally offset positions provide for partial longitudinal overlap of said first and second non-expanded portions in which parts thereof have the same longitudinal positions relative to said tube structure and other parts of said non-expanded portions have different longitudinal positions relative to said tube structure.

19. A vascular graft according to claim 17, wherein said expanded portion has a node (104) and fibril microstructure (106) in which nodes extend between said first and second non-expanded portions, said nodes having an inclined orientation relative-to a longitudinal plane of said PTFE tube structure subsequent to the formation of said expanded portion.

20. A vascular graft according to claim 17, and further comprising a plurality of first supplemental non-expanded portions (22, 96) formed from sintering the PTFE green tube extrudate, said first supplemental non-expanded portions being elongate and having a longitudinal central axis which is contained in said first longitudinal cross-sectional plane, said first supplemental and first non-expanded portions having the same longitudinal dimension and being of the same extrudate, said first supplemental and first non-expanded portions being separated longitudinally from adjacent ones of said first supplemental and first non-expanded portions by uniform dimensions,
said vascular graft further comprising a plurality of second supplemental non- expanded portions (98, 100) formed from sintering the PTFE green tube extrudate, said second supplemental non-expanded portions being elongate and having a longitudinal central axis which is contained in said second longitudinal cross-sectional plane, said second supplemental and second non-expanded portions having the same longitudinal dimensions and being of the same extrudate, said second supplemental and second non-expanded portions being separated longitudinally from adjacent ones of said first supplemental and first non-expanded portions by uniform dimensions which are the same as said uniform dimensions of said separation of said first supplemental and first non-expanded portions,
said first supplemental and first non-expanded portions being longitudinally symmetrical relative to said second supplemental and second non-expanded portions both before and after the formation of said expanded portion,
said dimension of said separation between said first supplemental and first non- expanded portions, and said dimension of said separation between said second supplemental and second non-expanded portions being increased during the formation of said expanded portion.

21. A vascular graft according to claim 20, wherein said expanded portion has a node (104) and fibril microstructure (106) in which nodes extend between said first and second non-expanded portions (22, 96, 98, 100), said node (104) and fibril microstructure having nodes which extend between said first and second supplemental non-expanded portions, said nodes which extend between said first and second non-expanded portions and said nodes which extend between said first and second supplemental non-expanded portions having an inclined orientation relative to a longitudinal plane of said PTFE tube structure subsequent to the formation of said expanded portion, said inclination of nodes (104) which extend between said first and second non-expanded portions being symmetrical relative to said inclination of nodes which extend between said first and second supplemental non-expanded portions about a transverse plane of said PTFE tube structure.

22. A vascular graft according to claim 1, wherein said non-expanded portion is oriented relative to a transverse cross-sectional plane of said PTFE tube structure such that said non-expanded portion provides a corresponding resistance to compression of said PTFE tube structure in said transverse cross-sectional plane.

23. A method for making a vascular graft according to any of claims 1 to 12, the method comprising the steps:
providing (32) a PTFE green tube extrudate (34) which is un-sintered; pre-sintering (36) a
section of the extrudate to produce a pre-sintered portion such that a section of the extrudate is un-sintered to constitute an un-sintered portion;
expanding (41) the un-sintered portion, said expansion of a region of the un-sintered portion which adjoins the pre-sintered portion being limited by the pre-sintered portion, the limiting of said expansion by the pre-sintered portion being attenuated in a region of the un- sintered portion which is remote from the pre-sintered portion.

24. A method according to claim 23, wherein the rate of said expansion is controlled such that said expansion of the section of the extrudate which contains the pre-sintered portion is different from said expansion of the region of the un-sintered portion which is remote from the pre-sintered portion.

## Patentansprüche

1. Gefäßprothese (10) zur Implantation in einen Körper, wobei die Gefäßprothese (10) aufweist:
eine PTFE-Schlauchstruktur (12) mit einer Längslänge und einer Innen- (14) und einer Außen- (16) Wandfläche sowie einem ersten und einem zweiten Längsteilstück (18, 20) an unterschiedlichen Längspositionen,
wobei die Schlauchstruktur (12) einen nichtexpandierten Abschnitt (22) im ersten Längsteilstück (18), der durch Sintern eines grünen/ungesinterten PTFE-Schlauchextrudats (34) gebildet ist, einen ersten expandierten Abschnitt (23) im ersten Längsteilstück (18) und einen durch das zweite Längsteilstück (20) gebildeten zweiten expandierten Abschnitt (24) hat, die beide im Anschluss an das Sintern gebildet werden,
der expandierte und nichtexpandierte Abschnitt (22) aus demselben Extrudat stammen,
der erste expandierte Abschnitt (23) einen Bereich hat, der an den nichtexpandierten Abschnitt (22) angrenzt, wobei ein Expansionsgrad des Bereichs durch den nichtexpandierten Abschnitt (22) begrenzt ist, wobei das Begrenzen der Expansion durch den nichtexpandierten Abschnitt (22) an einer Stelle des Bereichs abgeschwächt ist, die vom nichtexpandierten Abschnitt (22) entfernt ist,
wobei der nichtexpandierte Abschnitt (22) länglich ist und eine Längsmittelachse hat, die in einer Längsschnittebene (25) der PTFE-Schlauchstruktur (12) enthalten ist.

2. Gefäßprothese nach Anspruch 1, wobei der expandierte Abschnitt (23, 26) eine Knoten- und Fibrillenmikrostruktur hat.

3. Gefäßprothese nach Anspruch 1, wobei der nichtexpandierte Abschnitt (22) einen ersten nichtexpandierten Abschnitt aufweist, wobei die Gefäßprothese ferner drei zusätzliche nichtexpandierte Abschnitte (22) aufweist, die durch Sintern des grünen PTFE-Schlauchextrudats (34) gebildet sind, der erste nichtexpandierte und die drei zusätzlichen nichtexpandierten Abschnitte jeweils länglich sind und eine Längsmittelachse haben, die in jeweiligen Längsschnittebenen der PTFE-Schlauchstruktur (12) enthalten ist, die expandierten (23, 24) und der erste sowie die drei zusätzlichen nichtexpandierten Abschnitte (22) aus demselben Extrudat stammen, benachbarte Paare aus dem ersten und den zusätzlichen nichtexpandierten Abschnitten (22) relativ zur PTFE-Schlauchstruktur (12) um ein Winkelmaß gleich 90 Grad über den Umfang voneinander beabstandet sind, der erste und die zusätzlichen nichtexpandierten Abschnitte (22) jeweils ein jeweiliges proximales und distales Ende (26, 28) haben, die proximalen Enden die gleichen Längspositionen relativ zur PTFE-Schlauchstruktur haben und die distalen Enden die gleiche Längsposition relativ zur PTFE-Schlauchstruktur (12) haben,
der erste und die zusätzlichen nichtexpandierten Abschnitte im ersten Längsteilstück der PTFE-Schlauchstruktur (12) so enthalten sind, dass Einwirkung einer gleichmäßigen Längszugkraft auf die PTFE-Schlauchstruktur Längsdehnung des ersten Längsteilstücks und Längsdehnung des zweiten Längsteilstücks der PTFE-Schlauchstruktur (12) bewirkt, wobei die Längsdehnung des ersten Längsteilstücks kleiner als die Längsdehnung des zweiten Längsteilstücks ist.

4. Gefäßprothese nach Anspruch 3, wobei die gleichmäßige Längszugkraft bewirkt, dass die Längsdehnung des ersten Längsteilstücks 200 % beträgt und die Längsdehnung des zweiten Längsteilstücks 800 % beträgt.

5. Gefäßprothese nach Anspruch 1, wobei das Begrenzen der Expansion durch den nichtexpandierten Abschnitt (22) an Stellen des Teilstücks zunehmend abgeschwächt ist, die vom nichtexpandierten Abschnitt (22) zunehmend entfernt sind.

6. Gefäßprothese nach Anspruch 1, wobei ein weiterer nichtexpandierter Abschnitt (54) länglich ist und eine Längsmittelachse hat, die in einer Querschnittebene (56) der PTFE-Schlauchstruktur enthalten ist.

7. Gefäßprothese nach Anspruch 6, wobei der weitere nichtexpandierte Abschnitt (54) die Innenwandfläche so umfasst, dass der nichtexpandierte Abschnitt (54) ringförmig ist.

8. Gefäßprothese nach Anspruch 1, wobei ein weiterer nichtexpandierte Abschnitt (58, 60) länglich ist und eine Längsmittelachse hat, die relativ zu einer Querschnittebene (56) der PTFE-Schlauchstruktur geneigt ist.

9. Gefäßprothese nach Anspruch 8, wobei die Prothese einen ersten nichtexpandierten Abschnitt (58, 60) aufweist,
wobei die Gefäßprothese (10) einen zweiten nichtexpandierten Abschnitt aufweist, der durch Sintern des grünen PTFE-Schlauchextrudats gebildet ist, und der expandierte und zweite nichtexpandierte Abschnitt aus demselben Extrudat stammen,
der zweite nichtexpandierte Abschnitt (60, 58) länglich ist und eine Längsmittelachse hat, die relativ zu einer Querschnittebene (56) der PTFE-Schlauchstruktur (12) geneigt ist, die Neigung des zweiten nichtexpandierten Abschnitts entgegengesetzt zur Neigung des ersten nichtexpandierten Abschnitts ist und der erste und zweite nichtexpandierte Abschnitt einander schneiden.

10. Gefäßprothese nach Anspruch 1, wobei ein weiterer nichtexpandierter Abschnitt (64, 70) eine längliche Sägezahnkonfiguration hat.

11. Gefäßprothese nach Anspruch 10, wobei ein weiterer nichtexpandierter Abschnitt (64) eine Längshauptachse (66) hat, die die Sägezahnkonfiguration halbiert, wobei die Hauptachse (66) in einer Querschnittebene (68) der PTFE-Schlauchstruktur enthalten ist.

12. Gefäßprothese nach Anspruch 10, wobei ein weiterer nichtexpandierter Abschnitt (70) eine Längshauptachse (72) hat, die die Sägezahnkonfiguration halbiert, wobei die Hauptachse (72) in einer Längsschnittebene (74) der PTFE-Schlauchstruktur enthalten ist.

13. Gefäßprothese nach Anspruch 1, wobei die Gefäßprothese ferner nichtexpandierte Abschnitte aufweist, die aus einem nichtexpandierten Querabschnitt (76) gebildet sind, wobei die Gefäßprothese ferner einen weiteren nichtexpandierten Querabschnitt aufweist, der durch Sintern des grünen PTFE-Schlauchextrudats (34) gebildet ist, und der expandierte und nichtexpandierte Querabschnitt aus demselben Extrudat stammen,
die nichtexpandierten Querabschnitte (76) jeweils länglich sind und eine Längsmittelachse haben, die in einer entsprechenden Querschnittebene der PTFE-Schlauchstruktur enthalten ist, und die nichtexpandierten Querabschnitte (76) relativ zur PTFE-Schlauchstruktur längs voneinander getrennt sind,
wobei die nichtexpandierten Längsabschnitte (78) die durch Sintern des grünen PTFE-Schlauchextrudats gebildet sind, wobei die nichtexpandierten Längsabschnitte (78) jeweils länglich sind und eine Längsmittelachse haben, die in einer entsprechenden Längsschnittebene (25) der PTFE-Schlauchstruktur (12) enthalten ist, die expandierten und nichtexpandierten Längsabschnitte aus demselben Extrudat stammen und relativ zur PTFE-Schlauchstruktur (12) quer voneinander getrennt sind, und
die nichtexpandierten Längsabschnitte (78) die nichtexpandierten Querabschnitte schneiden.

14. Gefäßprothese nach Anspruch 1, wobei die Prothese ferner einen nichtexpandierten Abschnitt aufweist, der einen ersten nichtexpandierten Querabschnitt (84) bildet, wobei die Gefäßprothese ferner einen zweiten nichtexpandierten Querabschnitt (86) aufweist, der durch Sintern des grünen PTFE-Schlauchextrudats (34) gebildet ist, der erste und zweite nichtexpandierte Querabschnitt (84, 86) jeweils länglich sind und eine Längsmittelachse haben, die in einer entsprechenden Querschnittebene der PTFE-Schlauchstruktur (12) enthalten ist, der expandierte und der erste und zweite nichtexpandierte Querabschnitt aus demselben Extrudat stammen, der erste und zweite nichtexpandierte Querabschnitt relativ zur PTFE-Schlauchstruktur längs voneinander getrennt sind,
die Gefäßprothese (10) ferner einen ringförmigen nichtexpandierten Abschnitt (92, 94) aufweist, der durch Sintern des grünen PTFE-Schlauchextrudats (34) gebildet ist, der expandierte und ringförmige nichtexpandierte Abschnitt aus demselben Extrudat stammen und der ringförmige nichtexpandierte Abschnitt (92, 94) zwischen dem ersten und zweiten nichtexpandierten Querabschnitt in tangentialer Beziehung dazu liegt.

15. Gefäßprothese nach Anspruch 14, wobei der ringförmige nichtexpandierte Abschnitt (92, 94) einen ersten ringförmigen nichtexpandierten Abschnitt bildet, wobei die Gefäßprothese ferner einen dritten nichtexpandierten Querabschnitt (88) aufweist, der durch Sintern des grünen PTFE-Schlauchextrudats (34) gebildet ist, der dritte nichtexpandierte Querabschnitt länglich ist und eine Längsmittelachse hat, die in einer entsprechenden Querschnittebene der PTFE-Schlauchstruktur enthalten ist, der expandierte und dritte nichtexpandierte Querabschnitt aus demselben Extrudat stammen, der dritte nichtexpandierte Querabschnitt länglich ist und vom ersten und zweiten nichtexpandierten Querabschnitt relativ zur PTFE-Schlauchstruktur so längs getrennt ist, dass der dritte nichtexpandierte Querabschnitt vom ersten ringförmigen nichtexpandierten Abschnitt längs getrennt ist,
die Gefäßprothese ferner einen zweiten ringförmigen nichtexpandierten Abschnitt (92, 94) aufweist, der durch Sintern des grünen PTFE-Schlauchextrudats gebildet ist, der expandierte und zweite ringförmige nichtexpandierte Abschnitt aus demselben Extrudat stammen und der zweite ringförmige nichtexpandierte Abschnitt zwischen dem dritten nichtexpandierten Querabschnitt und dem ersten oder zweiten nichtexpandierten Querabschnitt in tangentialer Beziehung dazu liegt.

16. Gefäßprothese nach Anspruch 1, wobei die Prothese ferner einen nichtexpandierten Abschnitt aufweist, der eine Gitterstruktur aufweist.

17. Gefäßprothese nach Anspruch 1, wobei der nichtexpandierte Abschnitt (22, 96, 98, 100) einen ersten nichtexpandierten Abschnitt bildet, der länglich ist und eine Längsmittelachse hat, die in einer ersten Längsschnittebene der PTFE-Schlauchstruktur enthalten ist,
wobei die Gefäßprothese ferner einen zweiten nichtexpandierten Abschnitt aufweist, der durch Sintern des grünen PTFE-Schlauchextrudats gebildet ist, der zweite nichtexpandierte Abschnitt länglich ist und eine Längsmittelachse hat, die in einer zweiten Längsschnittebene der PTFE-Schlauchstruktur enthalten ist, der zweite nichtexpandierte Abschnitt vom ersten nichtexpandierten Abschnitt relativ zur PTFE-Schlauchstruktur über den Umfang getrennt ist und der expandierte und zweite nichtexpandierte Abschnitt aus demselben Extrudat stammen,
der erste und zweite nichtexpandierte Abschnitt längs versetzte Positionen relativ zueinander haben, wobei der erste und zweite nichtexpandierte Abschnitt während der Bildung des expandierten Abschnitts relativ zueinander längs verschoben werden.

18. Gefäßprothese nach Anspruch 17, wobei die längs versetzten Positionen eine teilweise Längsüberlappung des ersten und zweiten nichtexpandierten Abschnitts vorsehen, bei der Teile davon die gleichen Längspositionen relativ zur Schlauchstruktur haben und andere Teile der nichtexpandierten Abschnitte unterschiedliche Längspositionen relativ zur Schlauchstruktur haben.

19. Gefäßprothese nach Anspruch 17, wobei der expandierte Abschnitt eine Knoten-(104) und Fibrillenmikrostruktur (106) hat, in der sich Knoten zwischen dem ersten und zweiten nichtexpandierten Abschnitt erstrecken, wobei die Knoten eine geneigte Orientierung relativ zu einer Längsebene der PTFE-Schlauchstruktur im Anschluss an die Bildung des expandierten Abschnitts haben.

20. Gefäßprothese nach Anspruch 17 und ferner mit mehreren ersten ergänzenden nichtexpandierten Abschnitten (22, 96), die durch Sintern des grünen PTFE-Schlauchextrudats gebildet sind, wobei die ersten ergänzenden nichtexpandierten Abschnitte länglich sind und eine Längsmittelachse haben, die in der ersten Längsschnittebene enthalten ist, die ersten ergänzenden und ersten nichtexpandierten Abschnitte das gleiche Längsmaß haben und aus demselben Extrudat stammen, die ersten ergänzenden und ersten nichtexpandierten Abschnitte von benachbarten der ersten ergänzenden und ersten nichtexpandierten Abschnitte durch gleichmäßige Maße längs getrennt sind,
die Gefäßprothese ferner mehrere zweite ergänzende nichtexpandierte Abschnitte (98, 100) aufweist, die durch Sintern des grünen PTFE-Schlauchextrudats gebildet sind, die zweiten ergänzenden nichtexpandierten Abschnitte länglich sind und eine Längsmittelachse haben, die in der zweiten Längsschnittebene enthalten ist, die zweiten ergänzenden und zweiten nichtexpandierten Abschnitte die gleichen Längsmaße haben und aus demselben Extrudat stammen, die zweiten ergänzenden und zweiten nichtexpandierten Abschnitte von benachbarten der ersten ergänzenden und ersten nichtexpandierten Abschnitte durch gleichmäßige Maße längs getrennt sind, die die gleichen wie die gleichmäßigen Maße der Trennung der ersten ergänzenden und ersten nichtexpandierten Abschnitte, sind
die ersten ergänzenden und ersten nichtexpandierten Abschnitte relativ zu den zweiten ergänzenden und zweiten nichtexpandierten Abschnitten sowohl vor als auch nach der Bildung des expandierten Abschnitts längs symmetrisch sind, und das Maß der Trennung zwischen den ersten ergänzenden und ersten nichtexpandierten Abschnitten und das Maß der Trennung zwischen den zweiten ergänzenden und zweiten nichtexpandierten Abschnitten während der Bildung des expandierten Abschnitts zunehmen.

21. Gefäßprothese nach Anspruch 20, wobei der expandierte Abschnitt eine Knoten-(104) und Fibrillenmikrostruktur (106) hat, in der sich Knoten zwischen dem ersten und zweiten nichtexpandierten Abschnitt (22, 96, 98, 100) erstrecken, wobei die Knoten- (104) und Fibrillenmikrostruktur Knoten hat, die sich zwischen den ersten und zweiten ergänzenden nichtexpandierten Abschnitten erstrecken, die Knoten, die sich zwischen dem ersten und zweiten nichtexpandierten Abschnitt erstrecken, und die Knoten, die sich zwischen den ersten und zweiten ergänzenden nichtexpandierten Abschnitten erstrecken, eine geneigte Orientierung relativ zu einer Längsebene der PTFE-Schlauchstruktur im Anschluss an die Bildung des expandierten Abschnitts haben und die Neigung von Knoten (104), die sich zwischen dem ersten und zweiten nichtexpandierten Abschnitt erstrecken, relativ zur Neigung von Knoten, die sich zwischen den ersten und zweiten ergänzenden nichtexpandierten Abschnitten erstrecken, um eine Querebene der PTFE-Schlauchstruktur symmetrisch ist.

22. Gefäßprothese nach Anspruch 1, wobei der nichtexpandierte Abschnitt relativ zu einer Querschnittebene der PTFE-Schlauchstruktur so orientiert ist, dass der nichtexpandierte Abschnitt für einen entsprechenden Widerstand gegen Kompression der PTFE-Schlauchstruktur in der Querschnittebene sorgt.

23. Verfahren zur Herstellung einer Gefäßprothese nach einem der Ansprüche 1 bis 12, wobei das Verfahren die Schritte aufweist:
Bereitstellen (32) eines grünen PTFE-Schlauchextrudats (34), das ungesintert ist;
Vorsintern (36) eines Teilstücks des Extrudats, um einen vorgesinterten Abschnitt so herzustellen, dass ein Teilstück des Extrudats ungesintert ist, um einen ungesinterten Abschnitt zu bilden;
Expandieren (41) des ungesinterten Abschnitts, wobei die Expansion eines Bereichs des ungesinterten Abschnitts, der an den vorgesinterten Abschnitt angrenzt, durch den vorgesinterten Abschnitt begrenzt wird und das Begrenzen der Expansion durch den vorgesinterten Abschnitt in einem Bereich des ungesinterten Abschnitts abgeschwächt ist, der vom vorgesinterten Abschnitt entfernt ist.

24. Verfahren nach Anspruch 23, wobei die Rate der Expansion so gesteuert wird, dass sich die Expansion des Teilstücks des Extrudats, das den vorgesinterten Abschnitt enthält, von der Expansion des Bereichs des ungesinterten Abschnitts unterscheidet, der vom vorgesinterten Abschnitt entfernt ist.

## Revendications

1. Greffon vasculaire (10) destiné à être implanté dans un corps, ledit greffon vasculaire (10) comprenant:
une structure de tube en PTFE (12) ayant une longueur longitudinale et des surfaces de paroi interne (14) et externe (16) et des première et seconde sections longitudinales (18, 20) à différentes positions longitudinales,
ladite structure de tube (12) ayant une partie non expansée (22) dans la première section longitudinale (18) formée en frittant un extrudât de tube cru de PTFE (34), une première partie expansée (23) dans ladite première section longitudinale (18) et une seconde partie expansée (24) constituée par ladite seconde section longitudinale (20) toutes deux formées suite au frittage,
lesdites parties expansées et non expansée (22) étant réalisées avec le même extrudât,
ladite première partie expansée (23) ayant une région qui est attenante à ladite partie non expansée (22) dans lequel un degré d'expansion de ladite région est limitée par ladite partie non expansée (22), ladite limitation de ladite expansion par ladite partie non expansée (22) étant atténuée à un emplacement de ladite région qui est à distance de ladite partie non expansée (22),
dans lequel ladite partie non expansée (22) est allongée et a un axe central longitudinal contenu dans un plan transversal longitudinal (25) de ladite structure de tube en PTFE (12).

2. Greffon vasculaire selon la revendication 1, dans lequel ladite partie expansée (23, 26) a une microstructure de noeuds et de fibrilles.

3. Greffon vasculaire selon la revendication 1, dans lequel ladite partie non expansée (22) comprend une première partie non expansée, ledit greffon vasculaire comprenant trois parties non expansées supplémentaires (22) formées en frittant l'extrudât de tube cru en PTFE (34), ladite première partie non expansée et les trois parties non expansées supplémentaires étant chacune allongées et ayant un axe central longitudinal contenu dans des plans transversaux longitudinaux respectifs de ladite structure de tube en PTFE (12), lesdites parties expansées (23, 24) et la première et les trois parties non expansées supplémentaires (22) étant réalisées avec le même extrudât, des paires adjacentes desdites première partie et parties supplémentaires non expansées (22) étant séparées les unes des autres de manière circonférentielle par rapport à ladite structure de tube en PTFE (12) par une dimension angulaire égale à 90 degrés, lesdites première partie et parties supplémentaires non expansées (22) ayant chacune des extrémités proximales et distales (26, 28) respectives, lesdites extrémités proximales ayant les mêmes positions longitudinales par rapport à ladite structure de tube en PTFE, lesdites extrémités distales ayant la même position longitudinale par rapport à ladite structure de tube en PTFE (12),
lesdites première partie et parties non expansées supplémentaires étant contenues à l'intérieur de la première section longitudinale de ladite structure de tube en PTFE (12) de sorte que l'application d'une force de tension longitudinale uniforme sur ladite structure de tube en PTFE provoque l'allongement longitudinal de ladite première section longitudinale et l'allongement longitudinal de la seconde section longitudinale de ladite structure de tube en PTFE (12), l'allongement longitudinal de ladite première section longitudinale étant inférieur à l'allongement longitudinal de ladite seconde section longitudinale.

4. Greffon vasculaire selon la revendication 3, dans lequel la force de tension longitudinale uniforme provoque l'allongement longitudinal de ladite première section longitudinal de 200% et l'allongement longitudinal de ladite seconde section longitudinale de 800%.

5. Greffon vasculaire selon la revendication 1, dans lequel ladite limitation de ladite expansion par ladite partie non expansée (22) est progressivement atténuée aux emplacements de ladite section qui sont progressivement à distance de ladite partie non expansée (22).

6. Greffon vasculaire selon la revendication 1, dans lequel une autre partie non expansée (54) est allongée et a un axe central longitudinal contenu dans un plan transversal (56) de ladite structure de tube en PTFE.

7. Greffon vasculaire selon la revendication 6, dans lequel ladite autre partie non expansée (54) encercle ladite surface de paroi interne de sorte que ladite partie non expansée (54) est annulaire.

8. Greffon vasculaire selon la revendication 1, dans lequel une autre partie non expansée (58, 60) est allongée et a un axe central longitudinal qui est incliné par rapport à un plan transversal (56) de ladite structure de tube en PTFE.

9. Greffon vasculaire selon la revendication 8, dans lequel le greffon comprend une partie première non expansée (58, 60), ledit greffon vasculaire (10) comprenant une seconde partie non expansée formée en frittant l'extrudât de tube cru en PTFE, ladite partie expansée et ladite seconde partie non expansée étant réalisées avec le même extrudât,
ladite seconde partie non expansée (60, 58) étant allongée et ayant un axe central longitudinal qui est incliné par rapport à un plan transversal (56) de ladite structure de tube en PTFE (12), ladite inclinaison de ladite seconde partie non expansée étant opposée à ladite inclinaison de ladite première partie non expansée, lesdites première et seconde parties non expansées se coupant.

10. Greffon vasculaire selon la revendication 1, dans lequel une autre partie non expansée (64, 70) a une configuration en dents de scie allongée.

11. Greffon vasculaire selon la revendication 10, dans lequel une autre partie non expansée (64) a un axe principal longitudinal (66) qui coupe en deux parts égales ladite configuration en dents de scie, ledit axe principal (66) étant contenu dans un plan transversal (68) de ladite structure de tube en PTFE.

12. Greffon vasculaire selon la revendication 10, dans lequel une autre partie non expansée (70) a un axe principal longitudinal (72) qui coupe en deux parts égales ladite configuration en dents de scie, ledit axe principal (72) étant contenu dans un plan transversal longitudinal (74) de ladite structure de tube en PTFE.

13. Greffon vasculaire selon la revendication 1, dans lequel ledit greffon vasculaire comprend en outre des parties non expansées formées à partir d'une partie non expansée transversale (76), ledit greffon vasculaire comprenant en outre une autre partie non expansée transversale formée en frittant l'extrudât de tube cru en PTFE (34), lesdites parties expansées et non expansées transversales étant réalisées à partir du même extrudât,
lesdites parties non expansées transversales (76) étant chacune allongées et ayant un axe central longitudinal qui est contenu dans un plan transversal correspondant de ladite structure de tube en PTFE, lesdites parties non expansées transversales (76) étant séparées les unes des autres longitudinalement par rapport à ladite structure de tube en PTFE,
dans lequel lesdites parties non expansées longitudinales (78) sont formées en frittant l'extrudât de tube cru en PTFE, lesdites parties non expansées longitudinales (78) étant chacune allongées et ayant un axe central longitudinal qui est contenu dans un plan transversal longitudinal (25) correspondant de ladite structure de tube en PTFE (12), lesdites parties expansées et non expansées longitudinales étant réalisées à partir du même extrudât et séparées les unes des autres transversalement par rapport à ladite structure de tube en PTFE (12),
lesdites parties non expansées longitudinales (78) coupant lesdites parties non expansées transversales.

14. Greffon vasculaire selon la revendication 1, dans lequel ledit greffon comprend en outre une partie non expansée constituant une première partie non expansée transversale (84), ledit greffon vasculaire comprenant en outre une deuxième partie non expansée transversale (86) formée en frittant l'extrudât de tube cru en PTFE (34),
les première et seconde parties non expansées transversales (84, 86) étant chacune allongées et ayant un axe central longitudinal qui est contenu dans un plan transversal correspondant de la structure de tube en PTFE (12), lesdites parties expansées et les première et seconde parties non expansées transversales étant réalisées avec le même extrudât, lesdites première et seconde parties non expansées transversales étant séparées les unes des autres longitudinalement par rapport à ladite structure de tube en PTFE,
ledit greffon vasculaire (10) comprenant en outre une partie non expansée annulaire (92, 94) qui est formée en frittant l'extrudât de tube cru en PTFE (34), lesdites parties expansée et non expansée annulaire étant réalisées avec le même extrudât, ladite partie non expansée annulaire (92, 94) étant positionnée entre lesdites première et seconde parties non expansées transversales en relation tangentielle.

15. Greffon vasculaire selon la revendication 14, dans lequel ladite partie non expansée annulaire (92, 94) constitue une première partie non expansée annulaire, ledit greffon vasculaire comprenant en outre une troisième partie non expansée transversale (88) formée en frittant l'extrudât de tube cru en PTFE (34), ladite troisième partie non expansée transversale étant allongée et ayant un axe central longitudinal qui est contenu dans un plan transversal correspondant de ladite structure de tube en PTFE, lesdites parties expansées et la troisième partie non expansée transversale étant réalisées avec le même extrudât, ladite troisième partie non expansée transversale étant allongée et séparée desdites première et deuxième parties non expansées transversales longitudinalement par rapport à ladite structure de tube en PTFE de sorte que ladite troisième partie non expansée transversale est longitudinalement séparée de ladite première partie non expansée annulaire,
ledit greffon vasculaire comprenant en outre une seconde partie non expansée annulaire (92, 94) qui est formée en frittant l'extrudât de tube cru en PTFE, ladite partie expansée et ladite seconde partie non expansée annulaire étant réalisées avec le même extrudât, ladite seconde partie non expansée annulaire étant positionnée entre ladite troisième partie non expansée transversale et l'une desdites première et deuxième parties non expansées transversales en relation tangentielle.

16. Greffon vasculaire selon la revendication 1, dans lequel ledit greffon comprend en outre une partie non expansée comprenant une structure en treillis.

17. Greffon vasculaire selon la revendication 1, dans lequel ladite partie non expansée (22, 96, 98, 100) constitue une première partie non expansée qui est allongée et a un axe central longitudinal qui est contenu dans un premier plan transversal longitudinal de ladite structure de tube en PTFE,
ledit greffon vasculaire comprenant en outre une seconde partie non expansée formée en frittant l'extrudât de tube cru en PTFE, ladite seconde partie non expansée étant allongée et ayant un axe central longitudinal qui est contenu dans un second plan transversal longitudinal de ladite structure de tube en PTFE, ladite seconde partie non expansée étant séparée de ladite première partie non expansée circonférentiellement par rapport à ladite structure de tube en PTFE, ladite partie expansée et ladite seconde partie non expansée étant réalisées avec le même extrudât,
lesdites première et seconde parties non expansées ayant des positions longitudinalement décalées l'une par rapport à l'autre, dans lequel lesdites première et seconde parties non expansées sont longitudinalement déplacées l'une par rapport à l'autre pendant la formation de ladite partie expansée.

18. Greffon vasculaire selon la revendication 17, dans lequel lesdites parties longitudinalement décalées fournissent un chevauchement longitudinal partiel desdites première et seconde parties non expansées, dont leurs parties ont les mêmes positions longitudinales par rapport à ladite structure de tube et les autres parties desdites parties non expansées ont différentes positions longitudinales par rapport à ladite structure de tube.

19. Greffon vasculaire selon la revendication 17, dans lequel ladite partie expansée a une microstructure de noeuds (104) et de fibrilles (106) dans laquelle les noeuds s'étendent entre lesdites première et seconde parties non expansées, lesdits noeuds ayant une orientation inclinée par rapport à un plan longitudinal de ladite structure de tube en PTFE suite à la formation de ladite partie expansée.

20. Greffon vasculaire selon la revendication 17, et comprenant en outre une pluralité de premières parties non expansées supplémentaires (22, 96) formées en frittant l'extrudât de tube cru en PTFE, lesdites premières parties non expansées supplémentaires étant allongées et ayant un axe central longitudinal qui est contenu dans ledit premier plan transversal longitudinal, lesdites premières parties supplémentaires et lesdites premières parties non expansées ayant la même dimension longitudinale et étant réalisées avec le même extrudât, lesdites premières parties supplémentaires et lesdites premières parties non expansées étant séparées longitudinalement des parties adjacentes desdites premières parties supplémentaires et desdites premières parties non expansées par des dimensions uniformes,
ledit greffon vasculaire comprenant en outre une pluralité de secondes parties non expansées supplémentaires (98, 100) formées en frittant l'extrudât de tube cru en PTFE, lesdites secondes parties non expansées supplémentaires étant allongées et ayant un axe central longitudinal qui est contenu dans ledit second plan transversal longitudinal, lesdites secondes parties supplémentaires et lesdites secondes parties non expansées ayant les mêmes dimensions longitudinales et étant réalisées avec le même extrudât, lesdites secondes parties supplémentaires et lesdites secondes parties non expansées étant séparées longitudinalement des parties adjacentes desdites premières parties supplémentaires et desdites premières parties non expansées par des dimensions uniformes qui sont les mêmes que lesdites dimensions uniformes de ladite séparation desdites premières parties supplémentaires et desdites premières parties non expansées,
lesdites premières parties supplémentaires et lesdites premières parties non expansées étant longitudinalement symétriques par rapport auxdites secondes parties supplémentaires et auxdites secondes parties non expansées, avant et après la formation de ladite partie expansée,
ladite dimension de ladite séparation entre lesdites premières parties supplémentaires et lesdites premières parties non expansées, et ladite dimension de ladite séparation entre lesdites secondes parties supplémentaires et lesdites secondes parties non expansées étant augmentées pendant la formation de ladite partie expansée.

21. Greffon vasculaire selon la revendication 20, dans lequel ladite partie expansée a une microstructure de noeuds (104) et de fibrilles (106), dont des noeuds s'étendent entre lesdites première et seconde parties non expansées (22, 96, 98, 100), ladite microstructure de noeuds (104) et de fibrilles ayant des noeuds qui s'étendent entre lesdites première et seconde parties non expansées supplémentaires, lesdits noeuds qui s'étendent entre lesdites première et seconde parties non expansées et lesdits noeuds qui s'étendent entre lesdites première et seconde parties non expansées supplémentaires ayant une orientation inclinée par rapport à un plan longitudinal de ladite structure de tube en PTFE suite à la formation de ladite partie expansée, ladite inclinaison des noeuds (104) qui s'étendent entre lesdites première et seconde parties non expansées étant symétrique par rapport à ladite inclinaison des noeuds qui s'étendent entre lesdites première et seconde parties non expansées supplémentaires autour d'un plan transversal de ladite structure de tube en PTFE.

22. Greffon vasculaire selon la revendication 1, dans lequel ladite partie non expansée est orientée par rapport à un plan transversal de ladite structure de tube en PTFE de sorte que ladite partie non expansée fournit une résistance correspondante à la compression de ladite structure de tube en PTFE dans ledit plan transversal.

23. Procédé pour fabriquer un greffon vasculaire selon l'une quelconque des revendications 1 à 12, le procédé comprenant les étapes consistant à:
fournir (32) un extrudât de tube cru en PTFE (34) qui est non fritté; pré-fritter (36) une section de l'extrudât afin de produire une partie pré-frittée de sorte qu'une section de l'extrudât est non frittée pour constituer une partie non frittée;
expanser (41) la partie non frittée, ladite expansion d'une région de la partie non frittée qui est attenante à la partie pré-frittée étant limitée par la partie pré-frittée, la limitation de ladite expansion par la partie pré-frittée étant atténuée dans une région de la partie non frittée qui est à distance de la partie pré-frittée.

24. Procédé selon la revendication 23, dans lequel le taux de ladite expansion est contrôlé de sorte que ladite expansion de la section de l'extrudât qui contient la partie pré-frittée est différente de ladite expansion de la région de la partie pré-frittée qui est à distance de la partie pré-frittée.
